# EUROPEAN PATENT APPLICATION

(11) **EP 4 745 156 A1**
(43) Date of publication of application: **20.05.2026**
(21) Application number: 24842063.0
(22) Date of filing: 24.05.2024
(51) Int. Cl.: C07K 16/24, C12N 15/13, C12N 15/85, A61K 39/395, A61P 17/04

(54) **ANTIBODY CAPABLE OF BINDING TO INTERLEUKIN-31 OR ANTIGEN BINDING FRAGMENT THEREOF, AND PREPARATION METHOD THEREFOR AND USE THEREOF**

(30) Priority: 20.07.2023 CN 202310894799; 28.07.2023 CN 202310941457
(71) Applicant: Beijing Vjtbio Co., Ltd., Beijing 100085 (CN)
(72) Inventor: LUO, Haoshu, Beijing 100085 (CN); TAN, Zemin, Beijing 100085 (CN); YAN, Lin, Beijing 100085 (CN); WU, Bingchun, Beijing 100085 (CN); HAN, Guo, Beijing 100085 (CN)
(74) Representative: reuteler & cie SA
(86) International application number: PCT/CN2024/095251
(87) International publication number: WO 2025/016063

(57) **Abstract**

Provided are an antibody capable of binding to interleukin-31 or an antigen binding fragment thereof, and a preparation method therefor and a use thereof. The antibody or the antigen binding fragment thereof has high affinity and high activity, and by blocking the bingding of IL-31 and a receptor thereof, especially canine IL-31, the antibody or the antigen binding fragment thereof inhibits canine pruritic skin diseases, especially canine atopic dermatitis.

## Description

### CROSS-REFERENCE TO RELATED APPLICATIONS

The application claims priority to the China Application No. "202310894799.2", filed on July 20, 2023, entitled "ANTIBODY CAPABLE OF BINDING TO INTERLEUKIN-31 OR ANTIGEN BINDING FRAGMENT THEREOF, AND PREPARATION METHOD THEREFOR AND USE THEREOF", and the China Application No. "202310941457.1", filed on July 28, 2023, entitled "ANTIBODY CAPABLE OF BINDING TO INTERLEUKIN 31 OR ANTIGEN BINDING FRAGMENT THEREOF AS WELL AS PREPARATION METHOD AND APPLICATION OF ANTIBODY AND ANTIGEN BINDING FRAGMENT", the contents of which are specifically and entirely incorporated herein by reference.

### FIELD

The present disclosure relates to the field of antibodies, in particular to an antibody capable of binding to interleukin-31 or an antigen binding fragment thereof, and a preparation method and a use thereof.

### BACKGROUND

Skin diseases of dogs and cats are the most common diseases in pet hospitals, wherein the Canine Atopic Dermatitis (CAD) is a common pruritic skin disease in dogs. Canine Atopic Dermatitis can occur at any age, usually showing clinical symptoms in the summer on the dogs with an age around one year old, such as itchy, reddening, swollen and cracked skin. As the inflammation continues, the skin lesions become thicker. The causes of Canine Atopic Dermatitis may be associated with genetic diseases, immune system dysfunction, environmental exposure or skin permeability disorders.

Studies have discovered that transgenic mice overexpressing interleukin 31 (IL-31) develop symptoms such as skin inflammation and pruritus. In addition, an injection of IL-31 can induce acute pruritus behavior in mice. These studies show that IL-31 is closely related to skin inflammation and pruritus. IL-31 is mainly produced by activated helper Th2 cells, and IL-31 receptors are mainly expressed in macrophages, keratinocytes, and dorsal root ganglia. IL-31 receptors form heterodimers with IL-31RA and OSMR. After IL-31 binds to its receptor, it can activate the phosphorylation of downstream JAK kinases and STAT. In a mouse model of atopic dermatitis, IL-31 mRNA is highly expressed in the lesion site, and inhibiting the IL-31 signaling pathway can inhibit pruritus. Therefore, IL-31 antagonistic antibodies have the potential to inhibit pruritus behavior and relieve atopic dermatitis.

### SUMMARY

The present disclosure aims to overcome the defects in the prior art and provide an antibody capable of binding to interleukin-31 or an antigen binding fragment thereof, and a preparation method therefor and a use thereof.

### Definitions

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point value of the various ranges, and the individual point values may be combined with one another to produce one or more new numerical ranges, which should be deemed have been specifically disclosed herein.

In the present disclosure, the term "antibody" means an immunoglobulin molecule, and refers to any form of antibody that exhibits the desired biological activity, it includes but not limited to monoclonal antibodies (including full-length monoclonal antibodies), polyclonal antibodies and multi-specific antibodies (e.g., bispecific antibodies), and even antibody fragments. Typically, the full-length antibody structure preferably comprises four polypeptide chains, usually two heavy (H) chains and two light (L) chains interconnected by disulfide bonds. Each heavy chain comprises a heavy chain variable region and a heavy chain constant region. Each light chain comprises a light chain variable region and a light chain constant region. In addition to the typical full-length antibody structure, its structure may also include other derivative forms.

The term "antigen binding fragment" comprises a portion of an intact antibody molecule that retains at least some of the binding specificity of the parent antibody, typically including at least a portion of the antigen binding region or variable region (e.g., one or more CDRs) of the parent antibody. Examples of antigen binding fragment include but are not limited to Fv fragments, Fab fragments, Fab' fragments, Fab'-SH fragments, F(ab')2 fragments, Fd fragments, Fd' fragments, single-chain antibody molecules (e.g., scFv, di-scFv or tri-scFv, diabodies or scFab), single domain antibodies.

An "Fv fragment" contains both the heavy chain variable region and the light chain variable region, but lacks the constant region.

A "Fab fragment" consists of one light chain and the CH1 domain and variable regions of one heavy chain. The heavy chain of a Fab molecule cannot form a disulfide bond with another heavy chain molecule.

A "Fab' fragment" contains one light chain and a portion of one heavy chain including the VH domain and the CH1 domain and the region between the CHI domain and the CH2 domain, whereby an interchain disulfide bond can be formed between the two heavy chains of the two Fab' fragments to form a F(ab')2 molecule.

A "F(ab')2 fragment" contains two light chains and two heavy chains comprising a portion of the constant region between the CH1 domain and the CH2 domain, whereby an interchain disulfide bond is formed between the two heavy chains. Thus, a F(ab')2 fragment consists of two Fab' fragments held together by a disulfide bond between the two heavy chains.

"Single-chain Fv antibody" (or "scFv antibody") refers to an antibody fragment comprising the VH and VL domains of an antibody, wherein these domains are present in a single polypeptide chain. In general, the Fv polypeptide additionally comprises a polypeptide linker between the VH and VL domains, the linker enables the scFv to form the desired structure for antigen binding. For a review of scFv, please refer to Pluckthun (1994) THE PHARMACOLOGY OF MONOCLONAL ANTIBODIES, Vol. 113, editors-in-chief are Rosenburg and Moore, Springer-Verlag, New York, pp. 269-315. For further information, please refer to the International Patent Application Publication No. WO88/01649 and the patents US 4,946,778 and US 5,260,203. The contents of the above-mentioned documents are entirely incorporated herein by reference.

"Dual antibodies", also known as "bispecific antibodies", are small antibody fragments with two antigen binding sites. The fragments contain a heavy chain variable domain (VH) connected to a light chain variable domain (VL) in the same polypeptide chain (VH-VL or VL-VH). By using a linker that is too short to pair between the two domains on the same chain, the domains are forced to pair with the complementary domains of another chain and form two antigen binding sites.

The term "variable region" refers to the domain of an antibody heavy or light chain that is involved in the binding of the antibody to an antigen. The variable regions of the heavy and light chains of natural antibodies (VH and VL, respectively) generally have similar structures, and can be further subdivided into hypervariable regions (refer to complementarity determining regions (CDRs) interspersed with more conservative regions (refer to framework regions (FRs)). The term "complementarity determining regions" (CDRs, such as CDR1, CDR2, and CDR3) refers to the amino acid residues of an antibody variable region whose presence is essential for antigen binding. Each variable region typically has three CDR regions identified as CDR1, CDR2, and CDR3.

The term "constant region" refers to those amino acid sequences on the light and heavy chains of antibodies that are not directly involved in binding the antibody to an antigen, but exhibit various effector functions, such as antibody-dependent cytotoxicity.

The term "chimeric antibody" has at least a portion of the heavy chain variable region and at least a portion of the light chain variable region derived from one species; and at least a portion of the constant region derived from another species. For example, in one embodiment, a chimeric antibody can include a murine variable region and a canine constant region.

The term "canine-derived antibody" contains CDRs derived from non-canine antibodies, and framework regions and constant regions derived from canine antibodies. For example, the anti-IL31 antibodies provided herein may include CDRs derived from one or more murine antibodies and canine framework regions and constant regions. The present disclosure provides exemplary canine-derived antibodies. Additional anti-IL31 antibodies or variants thereof comprising heavy chain CDRs and light chain CDRs provided herein can be produced using any canine framework sequence and are also contained in the present disclosure. In one embodiment, the framework sequences suitable for use in the present disclosure include those framework sequences that are structurally similar to the framework sequences provided herein. Additional modifications may be made in the framework region to improve the properties of the antibodies provided herein. Such additional framework modifications may include chemical modifications; point mutations are performed to reduce immunogenicity or remove T cell epitopes; or mutations are performed and reversed to residues in the original germline sequence. In some embodiments, such modifications include those corresponding to the mutations exemplified herein, including reverse mutations to germline sequences. For example, in one embodiment, one or more amino acids in the canine framework region of the VH and/or VL of the canine-derived antibodies provided herein are subjected to reverse mutation to the corresponding amino acids in the parent murine antibody. For example, in the VH and VL of the canine-derived antibody, several sites of the framework amino acids of the template canine antibody are subjected to reverse mutation to the corresponding amino acid sequences in the mouse IL31 antibody. In one embodiment, in some embodiments, preferably one or more amino acid reverse mutations are selected from 2I, 4L, 36Y, 46L, 68R; more preferably, one or more amino acid reverse mutations are selected from 68G, 73F, 78L; further preferably, one or more amino acid reverse mutations are selected from 21, 12S, 37Q, 46V, 49K, 59P, 60S, 69S, 77S. Additional or alternative reverse mutations can be performed in the framework region of the canine-derived antibody provided herein to improve the properties of the antibody. The present disclosure also includes constant region mutations to improve the properties of antibodies. In some embodiments, the constant region of the canine IgG B heavy chain is mutated from M to A at site 234, from L to A at site 235, and from G to A at site 237 to reduce the affinity of canine IgG B with canine FcγRI and C1q, thereby avoiding the effector function of canine IgG B.

In the present disclosure, the term "Antibody Drug Conjugate" refers to a complex composed of a monoclonal antibody targeting a tumor-specific antigen or a tumor-associated antigen coupled with a different number of small molecule toxins via a connexon. It is a complex formed by connecting a cytotoxic drug to a monoclonal antibody targeting a tumor. After the Antibody Drug Conjugate molecules enter the body, they can bind to the antigens on the surface of the target cells through the guiding effect of the monoclonal antibody and enter the target cells. The Antibody Drug Conjugate molecules that enter the cells can release effector molecules through chemical and/or enzymatic action to achieve the purpose of eliminating the target cells.

In the present disclosure, the term "binding affinity" refers to the strength of the sum of non-covalent interactions between a single binding site of a molecule and its binding partner. Unless otherwise specified, "binding affinity" used herein refers to intrinsic binding affinity, which reflects a 1:1 interaction between members of a binding pair (e.g., an antibody and an antigen). The terms "Affinity KD", "association rate constant Kon", and "dissociation rate constant Koff" are commonly used to describe the affinity between a molecule (e.g., an antibody) and its binding partner (e.g., an antigen), i.e., the tightness with which a ligand binds to a specific protein. Binding affinity is affected by non-covalent intermolecular interactions, such as hydrogen bonds, electrostatic interactions, hydrophobic and Van der Waals forces between two molecules. In addition, the binding affinity between a ligand and its target molecule may be affected by the presence of other molecules.

In the present disclosure, the CDR numbering system in antibodies refers to the residues of the light and heavy chains that accurately define the complementarity determining regions (CDRs), frameworks (FRs), and influence the binding affinity and specificity of antibody-antigen interactions. Commonly used CDR numbering systems include Kabat, Chothia, IMGT, AbM, Contact, North, etc., and there may be differences when using different numbering systems.

In the present disclosure, the term "sequence identity" between two polypeptide or nucleic acid sequences refers to the percentage of the number of identical residues between the sequences relative to the total number of residues. When calculating the percentage of identity, the sequences being compared are aligned in a manner that produces the maximum match between the sequences, and gaps in the alignment (if any) are resolved by a specific algorithm. Preferred computer program methods for determining the identity between two sequences include, but are not limited to, the GCG program package, including GAP, BLASTP, BLASTN and FASTA (Altschul et al., 1990, J. Mol. Biol. 215: 403-410). The above programs can be publicly obtained from the International Center for Biotechnology Information (NCBI) and other sources. The well-known Smith Waterman algorithm can also be used to determine identity.

In the present disclosure, the cell line models are used on a cellular basis to study patient diseases, drug development and toxicity testing through changes in cells. In some embodiments of the present disclosure, HEK293 cells with the membrane expression of canine IL-31 receptors (canine IL-31RA and canine OSMR) are constructed to evaluate the ability of the IL31 antibody to block the binding of canine IL-31 recombinant protein to its receptor. In other embodiments, DH82 cells (canine macrophages/canine renal malignant histiocytosis cells) are used to study the IL31 antibody, block the binding of IL-31 with the cell surface co-receptors IL-31RA and OSMR, thereby inhibiting the downstream JAK/STAT signaling pathway and reducing the phosphorylation degree of STAT3.

In the present disclosure, animal models refer to different animals used as controllable experimental subjects for studying patient diseases, drug development and toxicity testing through different changes in animals; the commonly used animals are mammals, birds and reptiles. In some embodiments of the present disclosure, a canine pruritus model induced by recombinant canine IL31 is constructed to evaluate the inhibition of canine pruritus by the IL31 antibody. In other embodiments, a canine pruritus model induced by fleas is constructed to evaluate the inhibition of canine pruritus by the IL31 antibody.

In the present disclosure, the term "recombinant cell" refers to separating organelles and their components from living cells, and then recombining organelles and their components from different sources under certain conditions in vitro to reassemble them into cells or organelles with biological activity.

In the present disclosure, the term "cutaneous pruritus" refers to diseases mediated by IL-31, i.e., general inflammation; in particular, a series of diseases with dermatitis and pruritus symptoms, including atopic dermatitis, contact dermatitis, nodular pruritus, eczema and other allergic diseases.

The term "pharmaceutical composition" refers to a mixture containing one or more compounds of the present disclosure or its physiologically/pharmaceutically acceptable salt or prodrug and other chemical components, as well as other components such as physiologically/pharmaceutically acceptable carriers and excipients. The purpose of the pharmaceutical composition is to promote administration to a living organism, facilitate the absorption of the active ingredients and thereby exerting biological activity. The therapeutic composition should generally be sterile and stable under manufacturing and storage conditions. The composition can be formulated as a solution, micro-emulsion, dispersant, liposome or other ordered structure suitable for high antibody concentration. The active compound (i.e., antibody or antigen binding fragment thereof) can be incorporated in a desired amount together with one of the ingredients or combinations of ingredients listed above in a suitable solvent, and then filtered and sterilized as needed to prepare a sterile injectable solution.

In the present disclosure, the term "nucleotide molecule" is a biological macromolecular compound polymerized by many nucleotides, it is one of the most basic substances of life. Different nucleic acids have different chemical compositions, nucleotide arrangement sequences, etc. According to different chemical compositions, nucleic acids can be divided into ribonucleic acid (abbreviated as RNA) and deoxyribonucleic acid (abbreviated as DNA). DNA is the main material basis for storing, replicating and transmitting genetic information. RNA plays an important role in the protein synthesis process - among which transfer RNA (abbreviated as tRNA) plays the role of carrying and transferring activated amino acids; messenger RNA (abbreviated as mRNA) is the template for protein synthesis, and ribosomal RNA (abbreviated as rRNA) is the main site of protein synthesis in cells.

The first aspect of the present disclosure provides an antibody capable of binding to interleukin-31 or an antigen binding fragment thereof, which comprises one or more CDR region (antibody complementarity determining region) sequences selected from SEQ ID NOs: 1-6 and/or SEQ ID NOs: 7-12 and/or SEQ ID NOs: 13-18, or mutant sequences thereof.

In some embodiments, the IL31 antibody or the antigen binding fragment thereof described in the present disclosure comprises a heavy chain variable region CDR selected from SEQ ID NOs: 1-3 and/or SEQ ID NOs: 7-9 and/or SEQ ID NOs: 13-15, or a mutant sequence thereof.

In some embodiments, the IL31 antibody or antigen-binding fragment thereof described in the present disclosure comprises a light chain variable region CDR selected from SEQ ID NOs: 4-6 and/or SEQ ID NOs: 10-12 and/or SEQ ID NOs: 16-18, or a mutant sequence thereof.

In some embodiments, the IL31 antibody or the antigen binding fragment thereof described in the present disclosure comprises a heavy chain variable region CDR1, and amino acid sequence of the heavy chain variable region CDR1 has no less than about 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as set forth as SEQ ID NO: 1, 7 or 13.

In some embodiments, the IL31 antibody or the antigen binding fragment thereof described in the present disclosure comprises a heavy chain variable region CDR2, and amino acid sequence of the heavy chain variable region CDR2 has no less than about 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as set forth as SEQ ID NOs: 2, 8 and 14.

In some embodiments, the IL31 antibody or the antigen binding fragment thereof described in the present disclosure comprises a heavy chain variable region CDR3, and amino acid sequence of the heavy chain variable region CDR3 has no less than about 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as set forth as SEQ ID NOs: 3, 9 and 15.

In some embodiments, the IL31 antibody or the antigen binding fragment thereof described in the present disclosure comprises a light chain variable region CDR1, and amino acid sequence of the light chain variable region CDR1 has no less than about 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as set forth as SEQ ID NOs: 4, 10 and 16.

In some embodiments, the IL31 antibody or the antigen binding fragment thereof described in the present disclosure comprises a light chain variable region CDR2, and amino acid sequence of the light chain variable region CDR2 has no less than about 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as set forth as SEQ ID NOs: 5, 11 and 17.

In some embodiments, the IL31 antibody or the antigen binding fragment thereof described in the present disclosure comprises a light chain variable region CDR3, and amino acid sequence of the light chain variable region CDR3 has no less than about 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as set forth as SEQ ID NOs: 6, 12 and 18.

In some embodiments, for the IL31 antibody or the antigen binding fragment thereof described in the present disclosure, the amino acid sequence of heavy chain variable region CDR1 of the antibody or the antigen-binding fragment thereof has identity no lower than 80% with the amino acid sequence as set forth as SEQ ID NO: 1, 7 or 13, the amino acid sequence of heavy chain variable region CDR2 has identity no lower than 80% with the amino acid sequence as set forth as SEQ ID NOs: 2, 8 and 14, the amino acid sequence of heavy chain variable region CDR3 has identity no lower than 80% with the amino acid sequence as set forth as SEQ ID NOs: 3, 9 and 15, the amino acid sequence of light chain variable region CDR1 has identity no lower than 80% with the amino acid sequence as set forth as SEQ ID NOs: 4, 10 and 16, the amino acid sequence of light chain variable region CDR2 has identity no lower than 80% with the amino acid sequence as set forth as SEQ ID NOs: 5, 11 and 17, and the amino acid sequence of light chain variable region CDR3 has identity no lower than 80% with the amino acid sequence as set forth as SEQ ID NOs: 6, 12 and 18.

In the present disclosure, according to different antibody numbering systems, SEQ ID NO: 1 may also be SEQ ID NO: 19 or 25; SEQ ID NO: 7 may also be SEQ ID NO: 21 or 30, SEQ ID NO: 13 may also be SEQ ID NO: 23 or 35, SEQ ID NO: 2 may also be SEQ ID NO: 20 or 26, SEQ ID NO: 8 may also be SEQ ID NO: 22 or 31, SEQ ID NO: 14 may also be SEQ ID NO: 24 or 36, SEQ ID NO: 3 may also be SEQ ID NO: 27, SEQ ID NO: 9 may also be SEQ ID NO: 32, SEQ ID NO: 15 may also be SEQ ID NO: 37, SEQ ID NO: 4 may also be SEQ ID NO: 28, SEQ ID NO: 10 may also be SEQ ID NO: 33, SEQ ID NO: 16 may also be SEQ ID NO: 38, SEQ ID NO: 5 may also be SEQ ID NO: 29 (amino acid sequence is RA), SEQ ID NO: 11 may also be SEQ ID NO: 34, SEQ ID NO: 17 may also be SEQ ID NO: 39. Wherein SEQ ID NOs: 1-18 are CDRs obtained by using the Kabat numbering system, SEQ ID NOs: 3-6, 9-12 and 15-24 are CDRs obtained by using the Chothia numbering system, and SEQ ID NOs: 6, 12, 18 and 25-39 are CDRs obtained by using the IMGT numbering system. The amino acid sequence of SEQ ID NO: 34 is GA, and the amino acid sequence of SEQ ID NO: 39 is YV. That is, SEQ ID NO: 1 is substituted with SEQ ID NO: 19 or 25; SEQ ID NO: 7 is substituted with SEQ ID NO: 21 or 30, SEQ ID NO: 13 is substituted with SEQ ID NO: 23 or 35, SEQ ID NO: 2 is substituted with SEQ ID NO: 20 or 26, SEQ ID NO: 8 is substituted with SEQ ID NO: 22 or 31, SEQ ID NO: 14 is substituted with SEQ ID NO: 24 or 36, SEQ ID NO: 3 is substituted with SEQ ID NO: 27, SEQ ID NO: 9 is substituted with SEQ ID NO: 32, SEQ ID NO: 15 is substituted with SEQ ID NO: 37, SEQ ID NO: 4 is substituted with SEQ ID NO: 28, SEQ ID NO: 10 is substituted with SEQ ID NO: 33, SEQ ID NO: 16 is substituted with SEQ ID NO: 38, SEQ ID NO: 5 is substituted with SEQ ID NO: 29 (amino acid sequence is RA), SEQ ID NO: 11 is substituted with SEQ ID NO: 34 (amino acid sequence is GA), SEQ ID NO: 17 is substituted with SEQ ID NO: 39 (amino acid sequence is YV).

In the present disclosure, the number of amino acid residues in the heavy chain variable region CDR1 does not exceed 10; and/or, the number of amino acid residues in the heavy chain variable region CDR2 does not exceed 22; and/or, the number of amino acid residues in the heavy chain variable region CDR3 does not exceed 17; and/or, the number of amino acid residues in the light chain variable region CDR1 does not exceed 18; and/or, the number of amino acid residues in the light chain variable region CDR2 does not exceed 10; and/or, the number of amino acid residues in the light chain variable region CDR3 does not exceed 12.

In the present disclosure, in order to ensure that the obtained antibody or the antigen binding fragment thereof desirably inhibits IL-31 and has high affinity and high activity, thereby inhibiting canine pruritic skin diseases, the amino acid sequence of the heavy chain variable region CDR1 is as set forth as SEQ ID NO: 1, the amino acid sequence of the heavy chain variable region CDR2 is as set forth as SEQ ID NO: 2, the amino acid sequence of the heavy chain variable region CDR3 is as set forth as SEQ ID NO: 3, the amino acid sequence of the light chain variable region CDR1 is as set forth as SEQ ID NO: 4, the amino acid sequence of the light chain variable region CDR2 is as set forth as SEQ ID NO: 5, and the amino acid sequence of the light chain variable region CDR3 is as set forth as SEQ ID NO: 6.

In another embodiment of the present disclosure, the amino acid sequence of the heavy chain variable region CDR1 is as set forth as SEQ ID NO: 7, the amino acid sequence of the heavy chain variable region CDR2 is as set forth as SEQ ID NO: 8, the amino acid sequence of the heavy chain variable region CDR3 is as set forth as SEQ ID NO: 9, the amino acid sequence of the light chain variable region CDR1 is as set forth as SEQ ID NO: 10, the amino acid sequence of the light chain variable region CDR2 is as set forth as SEQ ID NO: 11, and the amino acid sequence of the light chain variable region CDR3 is as set forth as SEQ ID NO: 12.

In another embodiment of the present disclosure, the amino acid sequence of the heavy chain variable region CDR1 is as set forth as SEQ ID NO: 13, the amino acid sequence of the heavy chain variable region CDR2 is as set forth as SEQ ID NO: 14, the amino acid sequence of the heavy chain variable region CDR3 is as set forth as SEQ ID NO: 15, the amino acid sequence of the light chain variable region CDR1 is as set forth as SEQ ID NO: 16, the amino acid sequence of the light chain variable region CDR2 is as set forth as SEQ ID NO: 17, and the amino acid sequence of the light chain variable region CDR3 is as set forth as SEQ ID NO: 18.

In some embodiments, the antibodies or the antigen binding fragments thereof described in the present disclosure comprise a heavy chain variable region with an amino acid sequence selected from the group consisting of SEQ ID NOs: 40-43, 48-51, 56-59 and an amino acid sequence having at least about 80%, 85%, 90%, 95% identity; and a light chain variable region with an amino acid sequence selected from the group consisting of SEQ ID NOs: 44-47, 52-55, 60-63 and an amino acid sequence having at least about 80%, 85%, 90%, 95% identity.

In some embodiments, the antibody provided by the present disclosure is a chimeric antibody, and further provided is a chimeric anti-IL31 antibody, which comprises a heavy chain variable region and/or a light chain variable region.

Preferably, the antibody heavy chain variable region is selected from SEQ ID NOS: 40, 48, 56, and the antibody light chain variable region is selected from SEQ ID NOS: 44, 52, 60.

Preferably, the amino acid sequence of the heavy chain variable region of the chimeric anti-IL31 antibody is SEQ ID NO: 40 and an amino acid sequence with at least about 80%, 85%, 90%, or 95% identity, and the amino acid sequence of the light chain variable region is SEQ ID NO: 44 and an amino acid sequence with at least about 80%, 85%, 90%, or 95% identity.

Preferably, the amino acid sequence of the heavy chain variable region of the chimeric anti-IL31 antibody is SEQ ID NO: 48 and an amino acid sequence with at least about 80%, 85%, 90%, or 95% identity, and the amino acid sequence of the light chain variable region is SEQ ID NO: 52 and an amino acid sequence with at least about 80%, 85%, 90%, or 95% identity.

Preferably, the amino acid sequence of the heavy chain variable region of the chimeric anti-IL31 antibody is SEQ ID NO: 56 and an amino acid sequence with at least about 80%, 85%, 90%, or 95% identity, and the amino acid sequence of the light chain variable region is SEQ ID NO: 60 and an amino acid sequence with at least about 80%, 85%, 90%, or 95% identity.

Preferably, the heavy chain amino acid sequence is selected from SEQ ID NO: 66, 74 or 82; the light chain amino acid sequence is selected from SEQ ID NO: 70, 78 or 86.

More preferably, the antibody may have a heavy chain as set forth as SEQ ID NO: 66 and a light chain as set forth as SEQ ID NO: 70, or a heavy chain as set forth as SEQ ID NO: 74 and a light chain as set forth as SEQ ID NO: 78, or a heavy chain as set forth as SEQ ID NO: 82 and a light chain as set forth as SEQ ID NO: 86.

In the present disclosure, in order to enable the obtained antibody or the antigen binding fragment thereof to desirably inhibit IL-31 and have high affinity and high activity, thereby inhibiting canine pruritic skin diseases, the present disclosure provides a canine-derived antibody or an antigen binding fragment thereof.

In some embodiments, the heavy chain variable region framework FR amino acid sequence is selected from canine germline heavy chain sequences, preferably having 0-5 reverse mutation residues, such as 0, 1, 2, 3, 4 or 5 reverse mutations; in some embodiments, preferably one or more amino acid reverse mutations selected from the group consisting of 49G, 71V, 73K and 78A, and another preferably amino acid reverse mutations selected from the group consisting of 54A, 55E, 82D and 87V.

In some embodiments, the light chain variable region framework FR amino acid sequence is selected from canine germline light chain sequences, preferably having 0-5 reverse mutation residues, such as 0, 1, 2, 3, 4, or 5 back-mutant residues. In some embodiments, preferably one or more amino acid reverse mutations selected from the group consisting of 2I, 4L, 36Y, 46L and 68R; another preferably one or more amino acid reverse mutations selected from the group consisting of 68G, 73F and 78L; another preferably one or more amino acid reverse mutations selected from the group consisting of 2I, 12S, 37Q, 46V, 49K, 59P, 60S, 69S and 77S. The term "reverse mutation residue" refers to a residue that is completely or partially restored to its original genotype and phenotype after the second mutation of the amino acid residue.

In the present disclosure, the amino acid sequence of the heavy chain variable region is one of the amino acid sequences as set forth as SEQ ID NOS: 41-43, 49-51 and 57-59, and the amino acid sequence of the light chain variable region is one of the amino acid sequences as set forth as SEQ ID NOs: 45-47, 53-55 and 61-63.

In some embodiments, the present disclosure provides a canine-derived anti-IL31 antibody, comprising a heavy chain variable region having an amino acid sequence with at least 80%, 85%, 90%, 95% identity with the amino acid sequence as set forth as SEQ ID NOs: 41-43, and a light chain variable region having an amino acid sequence with at least 80%, 85%, 90%, 95% identity with the amino acid sequence as set forth as SEQ ID NOs: 45-47. Preferably, the amino acid sequence of the heavy chain variable region is one of the amino acid sequences as set forth as SEQ ID NOs: 41-43, and the amino acid sequence of the light chain variable region is one of the amino acid sequences as set forth as SEQ ID NOs: 45-47; more preferably, the amino acid sequence of the heavy chain variable region is one of the amino acid sequences as set forth as SEQ ID NOs: 42 and 43, and the amino acid sequence of the light chain variable region is one of the amino acid sequences as set forth as SEQ ID NOs: 45 and 47.

In some embodiments, the present disclosure provides a canine-derived anti-IL31 antibody, comprising a heavy chain variable region having an amino acid sequence with at least 80%, 85%, 90%, 95% identity with the amino acid sequence as set forth as SEQ ID NOs: 49-51, and a light chain variable region having an amino acid sequence with at least 80%, 85%, 90%, 95% identity with the amino acid sequence as set forth as SEQ ID NOs: 53-55. Preferably, the amino acid sequence of the heavy chain variable region is one of the amino acid sequences as set forth as SEQ ID NOs: 49-51, and the amino acid sequence of the light chain variable region is one of the amino acid sequences as set forth as SEQ ID NOs: 53-55; more preferably, the amino acid sequence of the heavy chain variable region is one of the amino acid sequences as set forth as SEQ ID NOs: 49 and 50, and the amino acid sequence of the light chain variable region is one of the amino acid sequences as set forth as SEQ ID NOs: 54 and 55.

In some embodiments, the present disclosure provides a canine-derived anti-IL31 antibody, comprising a heavy chain variable region having an amino acid sequence with at least 80%, 85%, 90%, 95% identity with the amino acid sequence as set forth as SEQ ID NOs: 57-59, and a light chain variable region having an amino acid sequence with at least 80%, 85%, 90%, 95% identity with the amino acid sequence as set forth as SEQ ID NOs: 61-63. Preferably, the amino acid sequence of the heavy chain variable region is one of the amino acid sequences as set forth as SEQ ID NOs: 57-59, and the amino acid sequence of the light chain variable region is one of the amino acid sequences as set forth as SEQ ID NOs: 61-63; more preferably, the amino acid sequence of the heavy chain variable region is one of the amino acid sequences as set forth as SEQ ID NOs: 58 and 59, and the amino acid sequence of the light chain variable region is one of the amino acid sequences as set forth as SEQ ID NOs: 61 and 62.

In some embodiments, the present disclosure provides the chimeric and/or canine-derived antibodies or antigen binding fragments, which comprise IgG A, IgG B, IgG C, IgG D heavy chain constant regions or variants thereof, preferably an IgG B mutant heavy chain constant region amino acid sequence composed of SEQ ID NO: 64; and a canine κ or λ chain light chain constant region or variants thereof, preferably the κ light chain constant region amino acid sequence composed of SEQ ID NO: 65.

In some embodiments, the present disclosure provides an anti-IL31 chimeric antibody, wherein the antibody comprises a complete heavy chain, and the heavy chain is selected from an amino acid sequence having at least about 90%, 91%, 92%, 93%, 94%, 95%, 96%, 97%, 98%, 99% identity with the amino acids consisting of SEQ ID NO: 66, 74 or 82; and the light chain amino acid sequence is selected from an amino acid sequence having at least about 90%, 91%, 92%, 93%, 94%, 95%, 96%, 97%, 98%, 99% identity with amino acids consisting of SEQ ID NO: 70, 78 or 86.

The chimeric antibody may be composed of a heavy chain as set forth as SEQ ID NO: 66 and a light chain as set forth as SEQ ID NO: 70; or a heavy chain as set forth as SEQ ID NO: 74 and a light chain as set forth as SEQ ID NO: 78; or a heavy chain as set forth as SEQ ID NO: 82 and a light chain as set forth as SEQ ID NO: 86.

In some embodiments, the present disclosure provides an anti-IL31 chimeric antibody, wherein the antibody comprises a complete heavy chain, and the heavy chain is selected from an amino acid sequence having at least about 90%, 91%, 92%, 93%, 94%, 95%, 96%, 97%, 98%, 99% identity with the amino acids consisting of SEQ ID NOs: 67-69, 75-77 and 83-85; and a complete light chain, the light chain amino acid sequence is selected from an amino acid sequence having at least about 90%, 91%, 92%, 93%, 94%, 95%, 96%, 97%, 98%, 99% identity with amino acids consisting of SEQ ID NOs: 71-73, 79-81 and 87-89.

The variable region of the canine-derived antibody is obtained by freely combining the canine-derived variable region heavy chain with the canine-derived variable region light chain of the same antibody. For example, the heavy chain 6465-H1 (SEQ ID NO: 41) can be combined with the light chain 6465-K1, 6465-K2 or 6465-K3 to form the variable regions labeled as 6465-H1K1, 6465-H1K2 and 6465-H1K3, respectively, and so on. For example, 6465-H1K1-canIgGB has a canine-derived variable region heavy chain H1, a canine-derived variable region light chain K1 and a canine IgGB constant region, and so on. The specific combinations are shown in Table 1.

In some embodiments, the antibody may be composed of a heavy chain with an amino acid sequence as set forth as SEQ ID NO: 67 and a light chain with an amino acid sequence as set forth as SEQ ID NO: 71, or a heavy chain with an amino acid sequence as set forth as SEQ ID NO: 68 and a light chain with an amino acid sequence as set forth as SEQ **ID** NO: 71, or a heavy chain with an amino acid sequence as set forth as SEQ ID NO: 69 and a light chain with an amino acid sequence as set forth as SEQ ID NO: 71, or a heavy chain with an amino acid sequence as set forth as SEQ ID NO: 67 and a light chain with an amino acid sequence as set forth as SEQ ID NO: 72, or a heavy chain with an amino acid sequence as set forth as SEQ ID NO: 68 and a light chain with an amino acid sequence as set forth as SEQ ID NO: 72, or a heavy chain with an amino acid sequence as set forth as SEQ ID NO: 69 and a light chain with an amino acid sequence as set forth as SEQ ID NO: 72, or a heavy chain with an amino acid sequence as set forth as SEQ ID NO: 67 and a light chain with an amino acid sequence as set forth as SEQ **ID** NO: 73, or a heavy chain with an amino acid sequence as set forth as SEQ ID NO: 68 and a light chain with an amino acid sequence as set forth as SEQ ID NO: 73, or a heavy chain with an amino acid sequence as set forth as SEQ ID NO: 69 and a light chain with an amino acid sequence as set forth as SEQ ID NO: 73, or a heavy chain with an amino acid sequence as set forth as SEQ ID NO: 75 and a light chain with an amino acid sequence as set forth as SEQ ID NO: 79, or a heavy chain with an amino acid sequence as set forth as SEQ ID NO: 76 and a light chain with an amino acid sequence as set forth as SEQ ID NO: 79, or a heavy chain with an amino acid sequence as set forth as SEQ ID NO:77 and a light chain with an amino acid sequence as set forth as SEQ **ID** NO:79, or a heavy chain with an amino acid sequence as set forth as SEQ **ID** NO:75 and a light chain with an amino acid sequence as set forth as SEQ ID NO:80, or a heavy chain with an amino acid sequence as set forth as SEQ ID NO:76 and a light chain with an amino acid sequence as set forth as SEQ ID NO:80, or a heavy chain with an amino acid sequence as set forth as SEQ ID NO:77 and a light chain with an amino acid sequence as set forth as SEQ ID NO:80, or a heavy chain with an amino acid sequence as set forth as SEQ ID NO:75 and a light chain with an amino acid sequence as set forth as SEQ ID NO:81, or a heavy chain with an amino acid sequence as set forth as SEQ **ID** NO:76 and a light chain with an amino acid sequence as set forth as SEQ ID NO:81, or a heavy chain with an amino acid sequence as set forth as SEQ **ID** NO:77 and a light chain with an amino acid sequence as set forth as SEQ ID NO:81, or a heavy chain with an amino acid sequence as set forth as SEQ ID NO:83 and a light chain with an amino acid sequence as set forth as SEQ ID NO:87, or a heavy chain with an amino acid sequence as set forth as SEQ ID NO:84 and a light chain with an amino acid sequence as set forth as SEQ ID NO:87, or a heavy chain with an amino acid sequence as set forth as SEQ ID NO:85 and a light chain with an amino acid sequence as set forth as SEQ ID NO:87, or a heavy chain with an amino acid sequence as set forth as SEQ **ID** NO:83 and a light chain with an amino acid sequence as set forth as SEQ ID NO: 88, or a heavy chain with an amino acid sequence as set forth as SEQ ID NO:84 and a light chain with an amino acid sequence as set forth as SEQ ID NO:88, or a heavy chain with an amino acid sequence as set forth as SEQ ID NO:85 and a light chain with an amino acid sequence as set forth as SEQ ID NO:88, or a heavy chain with an amino acid sequence as set forth as SEQ ID NO:83 and a light chain with an amino acid sequence as set forth as SEQ ID NO:89, or a heavy chain with an amino acid sequence as set forth as SEQ ID NO:84 and a light chain with an amino acid sequence as set forth as SEQ ID NO:89, or a heavy chain with an amino acid sequence as set forth as SEQ ID NO:85 and a light chain with an amino acid sequence as set forth as SEQ ID NO:89.

In the present disclosure, preferably, the antibody or the antigen binding fragment thereof is at least one selected from the group consisting of a monoclonal antibody (McAb), a polyclonal antibody, a multimeric antibody and a CDR grafting antibody; preferably, the antibody or the antigen binding fragment thereof is one selected from the group consisting of a single-chain antibody, a Fab antibody, a Fv antibody, a single-domain antibody, a (Fab)2 fragment, a scFv-Fc fusion protein, a scFv-Fv fusion protein, a Fv fragment and a minimum antigen recognition unit. The second aspect of the present disclosure provides a nucleotide molecule encoding the antibody or the antigen binding fragment thereof mentioned above.

The present disclosure is not limited to a specific nucleotide sequence, as long as the nucleotide sequences comply with the codon encoding rules and can be translated to obtain the aforementioned protein sequences, each of the nucleotide sequences falls into the protection scope of the present disclosure. If it is specifically limited, preferably, the nucleotide sequence of the DNA molecule encoding the heavy chain is one of SEQ ID NO: 90-93, 98-101 and 106-109 and its substantially similar sequence having at least 80%, 85%, 90%, 95%, 98%, 99% sequence identity therewith; another preferred nucleotide sequence of the DNA molecule encoding the light chain is one of SEQ ID NOs: 94-97, 102-105 and 110-113 and its substantially similar sequence having at least 80%, 85%, 90%, 95%, 98%, 99% sequence identity therewith.

More preferably, the DNA encoding the antibody may comprise: the heavy chain as set forth as SEQ ID NO: 90, and the light chain as set forth as SEQ ID NO: 94; the heavy chain as set forth as SEQ ID NO: 91, and the light chain as set forth as SEQ ID NO: 95; the heavy chain as set forth as SEQ ID NO: 92, and the light chain as set forth as SEQ ID NO: 95; the heavy chain as set forth as SEQ ID NO: 93, and the light chain as set forth as SEQ ID NO: 95; the heavy chain as set forth as SEQ ID NO: 91, and the light chain as set forth as SEQ ID NO: 96; the heavy chain as set forth as SEQ ID NO: 92, and the light chain as set forth as SEQ ID NO: 96; the heavy chain as set forth as SEQ ID NO: 93, and the light chain as set forth as SEQ ID NO: 96; the heavy chain as set forth as SEQ ID NO: 91, and the light chain as set forth as SEQ ID NO: 97; the heavy chain as set forth as SEQ ID NO: 92, and the light chain as set forth as SEQ ID NO: 97; the heavy chain as set forth as SEQ ID NO: 93, and the light chain as set forth as SEQ ID NO: 97; the heavy chain as set forth as SEQ ID NO: 98, and the light chain as set forth as SEQ ID NO: 102; the heavy chain as set forth as SEQ ID NO: ID NO:99, the light chain as set forth as SEQ ID NO:103; the heavy chain as set forth as SEQ ID NO:100, the light chain as set forth as shown in SEQ ID NO:103; the heavy chain as set forth as SEQ ID NO:101, the light chain as set forth as SEQ ID NO:103; the heavy chain as set forth as SEQ ID NO:99, the light chain as set forth as SEQ ID NO:104; the heavy chain as set forth as SEQ ID NO:100, the light chain as set forth as SEQ ID NO:104; the heavy chain as set forth as SEQ ID NO:101, the light chain as set forth as SEQ ID NO:104; the heavy chain as set forth as SEQ ID NO:99, the light chain is shown as set forth as SEQ ID NO:105; the heavy chain as set forth as SEQ ID NO:100, the light chain as set forth as SEQ ID NO:105; the heavy chain as set forth as SEQ ID NO:101, the light chain as set forth as SEQ ID NO:105; the heavy chain as set forth as SEQ ID NO:106, the light chain as set forth as SEQ ID NO:110; the heavy chain as set forth as SEQ ID NO:107, the light chain as set forth as SEQ ID NO:111; the heavy chain as set forth as SEQ ID NO: 108, and the light chain as set forth as SEQ ID NO: 111; the heavy chain as set forth as SEQ ID NO: 109, and the light chain as set forth as SEQ ID NO: 111; the heavy chain as set forth as SEQ ID NO: 107, and the light chain as set forth as SEQ ID NO: 112; the heavy chain as set forth as SEQ ID NO: 108, and the light chain as set forth as SEQ ID NO: 112; the heavy chain as set forth as SEQ ID NO: 109, and the light chain as set forth as SEQ ID NO: 112; the heavy chain as set forth as SEQ ID NO: 107, and the light chain as set forth as SEQ ID NO: 113; the heavy chain as set forth as SEQ ID NO: 108, and the light chain as set forth as SEQ ID NO: 113; the heavy chain as set forth as SEQ ID NO: 109, and the light chain as set forth as SEQ ID NO: 113.

The third aspect of the present disclosure provides an expression vector, wherein the expression vector expresses the aforementioned nucleotide molecule.

In the present disclosure, the expression vector refers to a vector that adds expression elements (e.g., promoter, RBS, terminator) to the basic skeleton of the cloning vector, so that the target gene can be expressed. In the present disclosure, the expression elements are added onto the nucleotides such that the expression vector can smoothly express the DNA molecule of the present disclosure, such as a plasmid containing the nucleotides of the present disclosure. Preferably, the expression vector is a eukaryotic expression vector, such as pCDNA3.1, pcDNA3.4.

The fourth aspect of the present disclosure provides a method for preparing an antibody or an antigen binding fragment thereof, wherein the method comprises transforming a host cell with the aforementioned expression vector.

In the present disclosure, the transformation method is not limited, and is a common method in the art of introducing an expression vector into a host cell for expression.

In the present disclosure, the host cell is not limited, as long as it can successfully express the nucleotide molecules of the present disclosure. Preferably, the host cell is a eukaryotic cell, more preferably a mammalian cell, including Chinese hamster ovary cells (CHO), NS0, NP2/0, Hela cells, 293T cells, HEK293 and other cell lines.

The fifth aspect of the present disclosure provides a pharmaceutical composition, wherein the pharmaceutical composition comprises an excipient and the aforementioned antibody or antigen binding fragment thereof or the aforementioned nucleotide molecule or expression vector. Preferably, the excipient is selected from a pharmaceutically acceptable carrier or excipient.

In the present disclosure, the pharmaceutical carrier and excipient are not limited as long as they are pharmaceutically acceptable, including but not limited to saline, buffer solution, glucose, water, glycerol, ethanol or a combination thereof.

The sixth aspect of the present disclosure provides a method for treating pruritic skin diseases in a mammal, wherein the method comprises: administering to the mammal the aforementioned antibody or the antigen binding fragment thereof, or the nucleic acid molecule, or the expression vector, or the pharmaceutical composition.

The antibody or the antigen binding fragment thereof is administered in an amount of 1-5 mg/kg, which can be 1 mg/kg, 2 mg/kg, 3 mg/kg, 4 mg/kg, 5 mg/kg or a random value within the range consisting of any two of the numerical values mentioned above.

The administration method includes but is not limited to intravenous injection, intramuscular injection, subcutaneous injection, sublingual administration, rectal perfusion, inhalation administration, and spray administration, and the like, more preferably subcutaneous injection. The pruritic skin diseases include, but are not limited to, atopic dermatitis, contact dermatitis, nodular pruritus and eczema, preferably atopic dermatitis.

The mammal is at least one selected from the group consisting of animals of *Felidae, Canidae, Suidae, Bovidae, Caprinae, Cercopithecidae, Muridae* and *Equidae* family; preferably, the mammal is at least one selected from the group consisting of a canine, a wolf, a jackal and a fox, more preferably a canine.

The seventh aspect of the present disclosure provides a use of the aforementioned antibody or the antigen binding fragment thereof, or the nucleic acid molecule, or the expression vector in the preparation of a medicament for treating a pruritic skin disease in a mammal.

The present disclosure provides the use of the aforementioned antibody or the antigen binding fragment thereof, or the nucleic acid molecule, or the expression vector in the treatment of a pruritic skin disease in a mammal.

The pruritic skin disease, the dosage and method of administering the drug, and the mammal are defined are previously mentioned, the content will not be repeatedly described herein.

Due to the technical scheme, the antibody or the antigen binding fragment thereof provided by the present disclosure has high affinity and high activity, and by blocking the binding of IL-31 and a receptor thereof, especially canine IL-31, the antibody or the antigen binding fragment thereof inhibits pruritic skin diseases of mammals.

### BRIEF DESCRIPTION OF THE DRAWINGS

FIG. 1 illustrates the APC positive cell rate in the presence of antibody 6465, 7475 or B2;
FIG. 2 illustrates the inhibitory effects of three chimeric antibodies and Lokivetmab on the canine IL-31 pruritus attack;
FIG. 3 illustrates the inhibitory effects of four canine-derived antibodies constructed based on the 6465 antibody on the canine IL-31 pruritus attack;
FIG. 4 illustrates the inhibitory effects of four canine-derived antibodies constructed based on the 7475 antibody on the canine IL-31 pruritus attack;
FIG. 5 illustrates the inhibitory effects of four canine-derived antibodies constructed based on the B2 antibody on the canine IL-31 pruritus attack.

### DETAILED DESCRIPTION

Example 1. Production and identification of mouse anti-canine interleukin 31 (IL-31) antibodies.

### (1) Antigen preparation

HEK293 cells were transiently transfected to express His-tagged canine IL-31 (interleukin 31) recombinant protein, the culture supernatant of the transiently transfected cells was collected and the supernatant was subjected to affinity chromatography by using a Ni column, and the eluent was the purified canine IL-31 recombinant protein.

### (2) Mouse immunization

Balb/c mice and C57 mice were immunized with canine IL-31 recombinant protein as antigen respectively. The antigen was emulsified with Freund's complete adjuvant for the first immunization, and 100µg of antigen was administered to each mouse. The antigen was emulsified with Freund's incomplete adjuvant for subsequent immunization, and 50µg of antigen was administered to each mouse. The interval was two weeks, and the injection method was intraperitoneal and subcutaneous multiple injections. After the fourth immunization, serum was collected and the antibody titer was analyzed by using enzyme-linked immunosorbent assay (ELISA), and the mice with serum immune titers exceeding 360,000 were selected for constructing an antibody library.

### (3) Spleen RNA extraction and reverse transcription

RNA from mouse spleen tissue was extracted by using the TRIzol method, and RNA integrity was identified by agarose gel electrophoresis. RNA concentration and OD₂₆₀/OD₂₈₀ were determined using an ultra-micro spectrophotometer. RNA with qualified quality was selected and reverse transcribed according to the specification of Superscript II reverse transcriptase (Thermo Scientific) to obtain cDNA.

### (4) Antibody library construction and phage display

The antibody heavy chain and light chain were amplified from cDNA to construct scFv antibody library and Fab antibody library respectively, with a storage capacity up to 1E8 cfu. The antibody library was electronically transferred to TG1 *Escherichia coli,* and the helper phage M13KO7 was then added to infect TG1 *Escherichia coli.* After overnight culture, the phage was precipitated with PEG/NaCl to prepare the phage display antibody library.

### (5) Phage display antibody library screening

The phage display antibody library was subjected to a solid phase screening and a cross-screening of solid phase and liquid phase by using the immunoassay tubes and the magnetic bead sorter respectively. For the solid phase screening, canine IL-31 recombinant protein was used to coat the immunoassay tubes, and for the liquid phase screening, biotinylated canine IL-31 recombinant protein was used to coat the Dynabeads magnetic beads. After three rounds of screening, Phage ELISA was used to identify the enriched phage collection and the monoclonal clones isolated from the collection.

### (6) Construction and expression purification of antibodies

DNA sequencing was performed on the phagemid of the Phage ELISA positive monoclonal clone, and the murine antibody variable region with the correct reading frame was selected from the sequencing results for gene synthesis, and the murine antibody variable region was then fused with the human IgG1 constant region and inserted into the pcDNA3.4 vector to construct a full-length antibody expression plasmid. The full-length antibody was transiently transfected and expressed in HEK293 cells, and the culture supernatant was purified by Protein A affinity chromatography to obtain the purified full-length antibody.

### (7) Screening of antibodies that block the binding of canine IL-31 to its receptor

The biotin-labeled canine IL-31 recombinant protein was incubated with antibodies of different concentrations for 5 minutes, then added into HEK293 cells with membrane-expressed canine IL-31 receptors (canine IL-31RA and canine OSMR), incubated on ice for 30 minutes, rinsed three times with PBS, and then added with streptavidin-APC fluorescent secondary antibody, and the proportion of cells labeled by APC was analyzed by using the flow cytometry. The APC-positive cell rate reflects the ability of biotin-labeled canine IL-31 recombinant protein to bind to cells with membrane-expressed canine IL-31 receptors. The lower the APC-positive cell rate, the stronger the ability of the antibody to block the binding of biotin-labeled canine IL-31 recombinant protein to its receptor.

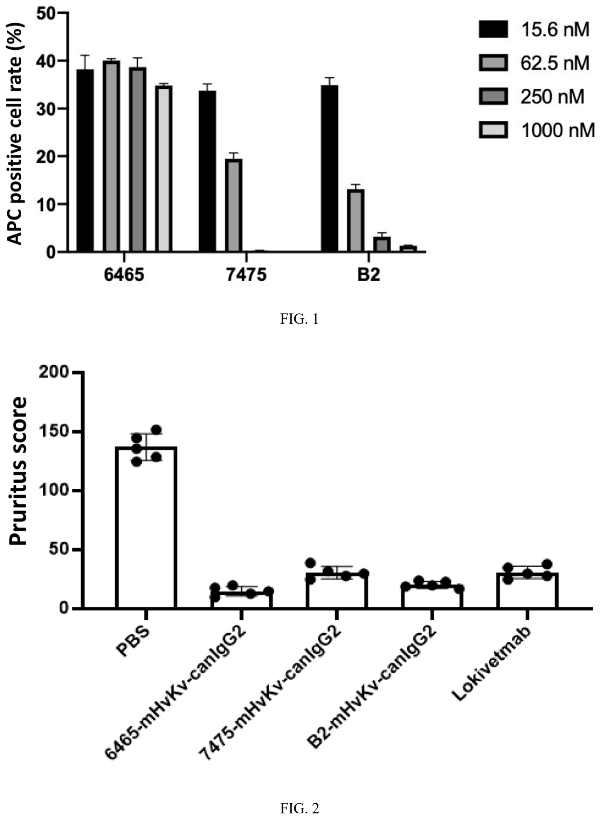

Results: The test antibodies 6465, 7475 and B2 can all lead to a decrease in APC positive cells as the antibody concentration increases, indicating that the antibodies have a blocking effect. The specific results are shown in FIG. 1. Using Kabat coding, the amino acid sequences of the heavy chain variable region complementarity determining regions (CDRs) 1, 2, and 3 of the antibody 6465 are SEQ ID NOs: 1-3, and the amino acid sequences of the light chain variable region CDRs 1, 2, and 3 are SEQ ID NOs: 4-6; the amino acid sequences of the heavy chain variable region CDRs 1, 2, and 3 of the antibody 7475 are SEQ ID NOs: 7-9, and the amino acid sequences of the light chain variable region CDRs 1, 2, and 3 are SEQ ID NOs: 10-12; the amino acid sequences of the heavy chain variable region CDRs 1, 2, and 3 of the antibody B2 are SEQ ID NOs: 13-15, and the amino acid sequences of the light chain variable region CDRs 1, 2, and 3 are SEQ ID NOs: 16-18. The amino acid sequences of the heavy chain variable region CDRs 1, 2 and 3 of antibody 6465 encoded using Chothia are SEQ ID NOs: 19, 20, and 3 respectively, and the amino acid sequences of the light chain variable region CDRs 1, 2 and 3 are SEQ ID NOs: 4-6 respectively; the amino acid sequences of the heavy chain variable region CDRs 1, 2 and 3 of antibody 7475 are SEQ ID NOs: 21, 22, and 9 respectively, and the amino acid sequences of the light chain variable region CDRs 1, 2 and 3 are SEQ ID NOs: 10-12 respectively; the amino acid sequences of the heavy chain variable region CDRs 1, 2 and 3 of antibody B2 are SEQ ID NOs: 23, 24, and 15 respectively, and the amino acid sequences of the light chain variable region CDRs 1, 2 and 3 are SEQ ID NOs: 16-18 respectively. Using IMGT encoding, the amino acid sequences of the heavy chain variable region CDRs 1, 2 and 3 of antibody 6465 are SEQ ID NOs: 25-27, and the amino acid sequences of the light chain variable region CDRs 1, 2 and 3 are SEQ ID NOs: 28, 29, and 6 respectively; the amino acid sequences of the heavy chain variable region CDRs 1, 2 and 3 of antibody 7475 are SEQ ID NOs: 30-32, and the amino acid sequences of the light chain variable region CDRs 1, 2 and 3 are SEQ ID NOs: 33, 34, and 12 respectively; the amino acid sequences of the heavy chain variable region CDRs 1, 2 and 3 of antibody B2 are SEQ ID NOs: 35-37, and the amino acid sequences of the light chain variable region CDRs 1, 2 and 3 are SEQ ID NOs: 38, 39, and 18 respectively.

### Example 2: Construction of chimeric and canine-derived antibodies of mouse anti-canine IL-31 antibodies

### (1) Chimeric antibodies

Canine chimeric antibody clones were constructed for the antibodies 6465, 7475 and B2, these chimeric antibodies had mouse heavy chain variable regions and mouse light chain variable regions. Based on the chimeric antibody of 6465 (6465-mHvKv-canIgG B), heavy chain variable region variants (SEQ ID NO: 40) and light chain variable region variants (SEQ ID NO: 44) were constructed; based on the chimeric antibody of 7475 (7475-mHvKv-canIgG B), heavy chain variable region variants (SEQ ID NO: 48) and light chain variable region variants (SEQ ID NO: 52) were constructed; based on the chimeric antibody of B2 (B2-mHvKv-canIgG B), heavy chain variable region variants (SEQ ID NO: 56) and light chain variable region variants (SEQ ID NO: 60), and the constant region (including CL, CH1, CH2, CH3) derived from canine IgG B antibody were constructed. The amino acid sequence of the constant region domain of canine IgG B heavy chain is as set forth as SEQ ID NO: 64. The amino acid sequence of the constant region domain of canine IgG B light chain is as set forth as SEQ ID NO: 65. The specific combination is shown in Table 1.

### (2) Canine-derived antibodies

1) Three canine-derived heavy chain variable region variants of the antibody 6465 (SEQ ID NOs: 41-43) and three canine-derived light chain variable region variants (SEQ ID NOs: 45-47) of the antibody 6465 were constructed, which contained various modifications or substitutions. Among them, the caninization strategies for 6465-H1 were CDR grafting and reverse mutations 49G, 71V and 73K, the caninization strategies for 6465-H2 were CDR grafting and reverse mutations 49G, 71V, 73K and 78A, and the caninization strategy for 6465-H3 was only CDR grafting. The caninization strategies for 6465-K1 were CDR grafting and reversion mutations 2I, 4L, 46L and 68R, the caninization strategies for 6465-K2 were CDR grafting and reverse mutations 4L, 36Y, 46L and 68R, and the caninization strategies for 6465-K3 were CDR grafting and reversion mutation 36Y.
2) Three canine-derived heavy chain variable region variants (SEQ ID NO: 49-51) and three canine-derived light chain variable region variants (SEQ ID NO: 53-55) of the antibody 7475 were constructed, which contained different modifications or substitutions. Among them, the caninization strategy for each of 7475-H1, 7475-H2 and 7475-H3 was only CDR grafting. The caninization strategy for each of 7475-K1 and 7475-K3 was only CDR grafting, while the caninization strategies for 7475-K2 were CDR grafting and reverse mutations 68G, 73F and 78L.
3) Three canine-derived heavy chain variable region variants (SEQ ID NO: 57-59) and three canine-derived light chain variable region variants (SEQ ID NO: 61-63) of the antibody B2 were constructed, which contained different modifications or substitutions. Among them, the caninization strategies of B2-H1 were CDR grafting and reverse mutations 54A, 82D and 87V; the caninization strategies of B2-H2 were CDR grafting and reverse mutations 55E, 82D and 87V; the caninization strategies of B2-H3 were CDR grafting and reverse mutations 55E, 82D and 87V. The caninization strategies for B2-K1 were CDR grafting and reverse mutations 2I, 46V, 49K and 69S, the caninization strategies for B2-K2 were CDR grafting and reverse mutations 46V, 49K and 69S, and the caninization strategies for B2-K3 were CDR grafting and reverse mutations 2I, 12S, 37Q, 46V, 49K, 59P, 60S, 69S and 77S.

The variable regions of the canine-derived antibody were formed by freely combining the canine-derived variable region heavy chain with the canine-derived variable region light chain of the same antibody, for example, the heavy chain 6465-H1 (SEQ ID NO: 41) can be combined with the light chain 6465-K1, 6465-K2 or 6465-K3, the formed variable regions were respectively labeled as 6465-H1K1, 6465-H1K2 and 6465-H1K3, and so on. The constant region of the canine-derived antibody was composed of the constant domain of the canine IgG B heavy chain and the constant domain of the canine Kappa light chain (the amino acid sequence of the constant region domain of the canine IgG B heavy chain was as set forth as SEQ ID NO: 64, the amino acid sequence of the constant region domain of the canine Kappa light chain was as set forth as SEQ ID NO: 65), for example, 6465-H1K1-canIgG B had a canine-derived variable region heavy chain H1, a canine-derived variable region light chain K1 and a canine IgG B constant region, and so on, and the specific combinations were shown in Table 1.

The constant region of the canine IgG B heavy chain contained three amino acid mutations, namely M234A, L235A and G237A, the purpose of which was to reduce the affinity of canine IgG B with canine FcγRI and C1q, such that the effector function of canine IgG B was avoided.

**Table 1 Sequence listing of chimeric antibodies and canine-derived antibody**

| Types | Antibody names | Variable region SEQ ID NO: | | Constant region SEQ ID NO: | |
|---|---|---|---|---|---|
| | | VH (heavy chain) | VL (light chain) | VH (heavy chain) | VL (light chain) |
| Chimeric antibody based on 6465 | 6465-mHvKv-canIgGB | 40 | 44 | 64 | 65 |
| Canine-derived antibody based on 6465 | 6465-H1K1-canIgGB | 41 | 45 | 64 | 65 |
| | 6465-H2K1-canIgGB | 42 | 45 | 64 | 65 |
| | 6465-H3K1-canIgGB | 43 | 45 | 64 | 65 |
| | 6465-H1K2-canIgGB | 41 | 46 | 64 | 65 |
| | 6465-H2K2-canIgGB | 42 | 46 | 64 | 65 |
| | 6465-H3K2-canIgGB | 43 | 46 | 64 | 65 |
| | 6465-H1K3-canIgGB | 41 | 47 | 64 | 65 |
| | 6465-H2K3-canIgGB | 42 | 47 | 64 | 65 |
| | 6465-H3K3-canIgGB | 43 | 47 | 64 | 65 |
| Chimeric antibody based on 7475 | 7475-mHvKv-canIgGB | 48 | 52 | 64 | 65 |
| Canine-derived antibody based on 7475 | 7475-H1K1-canIgGB | 49 | 53 | 64 | 65 |
| | 7475-H2K1-canIgGB | 50 | 53 | 64 | 65 |
| | 7475-H3K1-canIgGB | 51 | 53 | 64 | 65 |
| | 7475-H1K2-canIgGB | 49 | 54 | 64 | 65 |
| | 7475-H2K2-canIgGB | 50 | 54 | 64 | 65 |
| | 7475-H3K2-canIgGB | 51 | 54 | 64 | 65 |
| | 7475-H1K3-canIgGB | 49 | 55 | 64 | 65 |
| | 7475-H2K3-canIgGB | 50 | 55 | 64 | 65 |
| | 7475-H3K3-canIgGB | 51 | 55 | 64 | 65 |
| Chimeric antibody based on B2 | B2-mHvKv-canIgGB | 56 | 60 | 64 | 65 |
| Canine-derived antibody based on B2 | B2-H1K1-canIgGB | 57 | 61 | 64 | 65 |
| | B2-H2K1-canIgGB | 58 | 61 | 64 | 65 |
| | B2-H3K1-canIgGB | 59 | 61 | 64 | 65 |
| | B2-H1K2-canIgGB | 57 | 62 | 64 | 65 |
| | B2-H2K2-canIgGB | 58 | 62 | 64 | 65 |
| | B2-H3K2-canIgGB | 59 | 62 | 64 | 65 |
| | B2-H1K3-canIgGB | 57 | 63 | 64 | 65 |
| | B2-H2K3-canIgGB | 58 | 63 | 64 | 65 |
| | B2-H3K3-canIgGB | 59 | 63 | 64 | 65 |

### Example 3. In vitro evaluation of chimeric antibodies and canine-derived antibodies

### (1) In vitro activity evaluation of new antibodies constructed based on the antibodies 6465, 7475 and B2

Inhibition measurement of canine IL-31-induced STAT (signal transducer and activator of transcription) phosphorylation by new antibodies:
IL-31 activated the downstream JAK/STAT signaling pathway by binding to its cell surface co-receptors IL-31RA and OSMR, resulting in an increased STAT3 phosphorylation degree. When IL-31 neutralizing antibodies blocked the binding of IL-31 to its receptor, STAT3 phosphorylation in its downstream signaling pathway was also inhibited. The anti-canine IL-31 chimeric antibodies and canine-derived antibodies with different concentration gradients were used to evaluate the IC50 value of antibody inhibition of STAT phosphorylation in DH82 cell assays. The specific process was as follows: 50,000 DH82 cells were disposed at each pore, 10 ng/mL IFN-γ was added, stimulated at 37°C for 24 hours, and then subjected to serum starvation for 2 hours. After incubation of canine IL-31 recombinant protein having a concentration of 2.86 µg/mL with canine IL-31 antibodies having different concentrations for 1 hour, the above-mentioned DH82 cells were added and stimulated at 37°C for 5 minutes. The STAT3 phosphorylation level of DH82 cells was then detected with reference to the specification of the HTRF ^{®} STAT3 p-Y705 kit, and the IC50 value was calculated according to the antibody concentration. The results were shown in Table 2, The 3 chimeric antibodies and 27 canine-derived antibodies were able to inhibit the activity of pSTAT, IC50 value was within the range of 6.7-9.4 µg/mL.

**Table 2 Activity of canine-derived antibodies to induce pSTAT**

| Anti-canine IL-31 antibody | pSTAT IC50 (µg/mL) | Anti-canine IL-31 antibody | pSTAT IC50 (µg/mL) | Anti-canine IL-31 antibody | pSTAT IC50 (µg/mL) |
|---|---|---|---|---|---|
| 6465-mHvKv-canIgGB | 7.1 | 7475-mHvKv-canIgGB | 6.7 | B2-mHvKv-canIgGB | 6.8 |
| 6465-H1K1-canIgGB | 7.5 | 7475-H1K1-canIgGB | 8.4 | B2-H1K1-canIgGB | 9.4 |
| 6465-H2K1-canIgGB | 7.3 | 7475-H2K1-canIgG B | 7.9 | B2-H2K1-canIgG B | 8.3 |
| 6465-H3K1-canIgGB | 7.8 | 7475-H3K1-canIgGB | 7.5 | B2-H3K1-canIgGB | 8.5 |
| 6465-H1K2-canIgGB | 7.9 | 7475-H1K2-canIgGB | 8.1 | B2-H1K2-canIgGB | 9.2 |
| 6465-H2K2-canIgGB | 7.3 | 7475-H2K2-canIgGB | 7.1 | B2-H2K2-canIgGB | 7.8 |
| 6465-H3K2-canIgGB | 8.2 | 7475-H3K2-canIgGB | 7.8 | B2-H3K2-canIgGB | 7.2 |
| 6465-H1K3-canIgGB | 7.4 | 7475-H1K3-canIgGB | 8.2 | B2-H1K3-canIgGB | 9.3 |
| 6465-H2K3-canIgGB | 7.6 | 7475-H2K3-canIgGB | 7.2 | B2-H2K3-canIgGB | 7.1 |
| 6465-H3K3-canIgGB | 7.2 | 7475-H3K3-canIgGB | 6.9 | B2-H3K3-canIgGB | 7.4 |

### (2) Affinity measurement of new antibodies constructed based on the antibodies 6465, 7475 and B2

The binding affinity of the antibodies was measured by using the Gator instrument based on the Biofilm Lateral Interference (BLI) technology. When the solid on the biosensor interacted with the analyte in the solution, the thickness of the biological film layer on the sensor increases, and the interference spectrum curve moved in the direction of increasing wavelength. The phase shift was detected and analyzed by the workstation, and the changes in the number of molecules on the sensor surface and related concentration and kinetic data can be quantitatively obtained, such as the dissociation rate K_{off}, the association rate Kₒₙ and the affinity KD, where KD = K_{off} /Kₒₙ .

The binding affinity of 3 chimeric antibodies, 27 canine-derived antibodies and the positive control Lokivetmab was tested respectively, the specific process was as follows: the antibody was first biotin labelled, and the biotin labelled antibody was then used as a condensate to bind with an antibody using a streptavidin probe (Gator^{™} Streptavidin Probes) to solidify the biotin label. Subsequently, the canine IL-31 recombinant protein with different concentration gradients was used as an analyte to measure its binding kinetic data with the biotin labelled antibody and calculate the affinity. The results were shown in Table 3, the binding affinity KD of the 3 chimeric antibodies and the 27 canine-derived antibodies reached 10⁻⁹M, which was about 10 times of the affinity of positive control Lokivetmab.

**Table 3 Affinity data of IL31 antibodies**

| Anti-canine IL-31 antibodies | Dissociation rate Koff (1/s) | Association rate Kon (1/Ms) | Affinity KD (M) |
|---|---|---|---|
| 6465-mHvKv-canIgGB | 1.06E-03 | 5.07E+05 | 2.09E-09 |
| 6465-H1K1-canIgGB | 9.79E-04 | 3.05E+05 | 3.21E-09 |
| 6465-H2K1-canIgGB | 1.71E-03 | 5.08E+05 | 3.37E-09 |
| 6465-H3K1-canIgGB | 1.21E-03 | 2.11E+05 | 5.75E-09 |
| 6465-H1K2-canIgGB | 8.80E-04 | 1.35E+05 | 6.54E-09 |
| 6465-H2K2-canIgGB | 1.66E-03 | 9.32E+04 | 1.78E-08 |
| 6465-H3K2-canIgGB | 3.61E-03 | 5.19E+05 | 6.95E-09 |
| 6465-H1K3-canIgGB | 1.45E-03 | 5.79E+05 | 2.50E-09 |
| 6465-H2K3-canIgGB | 1.00E-03 | 2.01E+05 | 4.99E-09 |
| 6465-H3K3-canIgGB | 1.64E-03 | 8.12E+05 | 2.02E-09 |
| 7475-mHvKv-canIgGB | 2.51E-04 | 2.50E+05 | 1.01E-09 |
| 7475-H1K1-canIgGB | 1.56E-03 | 2.31E+05 | 6.73E-09 |
| 7475-H2K1-canIgGB | 5.50E-04 | 1.01E+05 | 5.43E-09 |
| 7475-H3K1-canIgGB | 5.42E-04 | 1.12E+05 | 4.84E-09 |
| 7475-H1K2-canIgGB | 6.14E-04 | 9.65E+04 | 6.37E-09 |
| 7475-H2K2-canIgGB | 4.81E-04 | 1.33E+05 | 3.61E-09 |
| 7475-H3K2-canIgGB | 5.39E-04 | 1.00E+05 | 5.40E-09 |
| 7475-H1K3-canIgGB | 1.92E-03 | 2.95E+05 | 6.51E-09 |
| 7475-H2K3-canIgGB | 7.59E-04 | 1.62E+05 | 4.67E-09 |
| 7475-H3K3-canIgGB | 2.04E-03 | 6.11E+05 | 3.34E-09 |
| B2-mHvKv-canIgGB | 2.43E-04 | 7.99E+04 | 3.04E-09 |
| B2-H1K1-canIgGB | 1.30E-03 | 7.56E+04 | 1.72E-08 |
| B2-H2K1-canIgGB | 9.42E-04 | 9.38E+04 | 1.00E-08 |
| B2-H3K1-canIgGB | 1.14E-03 | 1.12E+05 | 1.02E-08 |
| B2-H1K2-canIgGB | 1.15E-03 | 8.28E+04 | 1.38E-08 |
| B2-H2K2-canIgGB | 8.82E-04 | 9.62E+04 | 9.17E-09 |
| B2-H3K2-canIgGB | 1.88E-04 | 1.07E+05 | 1.76E-09 |
| B2-H1K3-canIgGB | 1.50E-03 | 8.98E+04 | 1.67E-08 |
| B2-H2K3-canIgGB | 1.43E-04 | 9.35E+04 | 1.53E-09 |
| B2-H3K3-canIgGB | 3.81E-04 | 1.21E+05 | 3.16E-09 |
| Lokivetmab | 2.03E-03 | 1.05E+05 | 1.93E-08 |

| | | | |
|---|---|---|---|
| Note: E denotes "×10" in the scientific notation, the numerical value behind E refers to a power of 10. | | | |

### Example 4. In vivo pharmacodynamic evaluation of chimeric antibodies and canine-based antibodies based on the antibodies 6465, 7475 and B2

The in vivo pharmacodynamic evaluation of anti-canine IL-31 chimeric antibodies and canine-derived antibodies was performed by using the canine IL-31 pruritus challenge model.

First, the beagles (with a weight about 10 kg) were modeled and screened for suitable experimental dogs. The canine IL-31 recombinant protein was injected intravenously according to a criterion of 2.5 µg/kg, and the pruritus behaviors of beagles (such as chewing, scratching, biting, licking, rolling and rubbing) in half an hour before the administration and four hours after the administration were monitored by video. If the pruritus behaviors were observed within 1 minute, 1 point was scored, and if not, 0 point was scored. Scoring was performed with an interval of 1 minute. The cumulative pruritus score half an hour before the administration was used as the baseline value, and the cumulative pruritus score four hours after the administration was used as the measured value. The measured value was deducted by its baseline value to obtain the final pruritus score. Due to individual differences, the pruritus score of each beagle after modeling fluctuated greatly. For ensuring the model uniformity, the beagles with pruritus scores between 100-150 points were screened and grouped.

Beagles with successful modeling were screened, and 25 dogs were selected and randomly divided into 5 groups, which included 3 drug-administered groups (6465, 7475 and B2 chimeric antibodies), a PBS control group and a positive control group (Lokivetmab), with 5 dogs in each group. After 7 days of adaptation, the beagles in the drug-administered groups were subcutaneously injected with 2 mg/kg 6465, 7475 and B2 chimeric antibodies, the beagles in the positive control group were subcutaneously injected with 2 mg/kg Lokivetmab, and the negative control PBS group was subcutaneously injected with an equal volume of PBS. On the next day, all beagles were intravenously injected with 2.5 µg/kg canine IL-31 recombinant protein. According to the above scoring criteria, the pruritus score of each group of beagles was recorded to evaluate whether the drug-administered antibodies inhibited IL-31 mediated pruritus. The results were shown in FIG. 2. The Administration of PBS to beagles could not reduce the pruritus symptoms caused by IL31, and the pruritus score was still between 100 and 150. In contrast, the administration of three chimeric antibodies and Lokivetmab could significantly reduce the pruritus caused by IL31 in beagles with an obviously inhibitory effect.

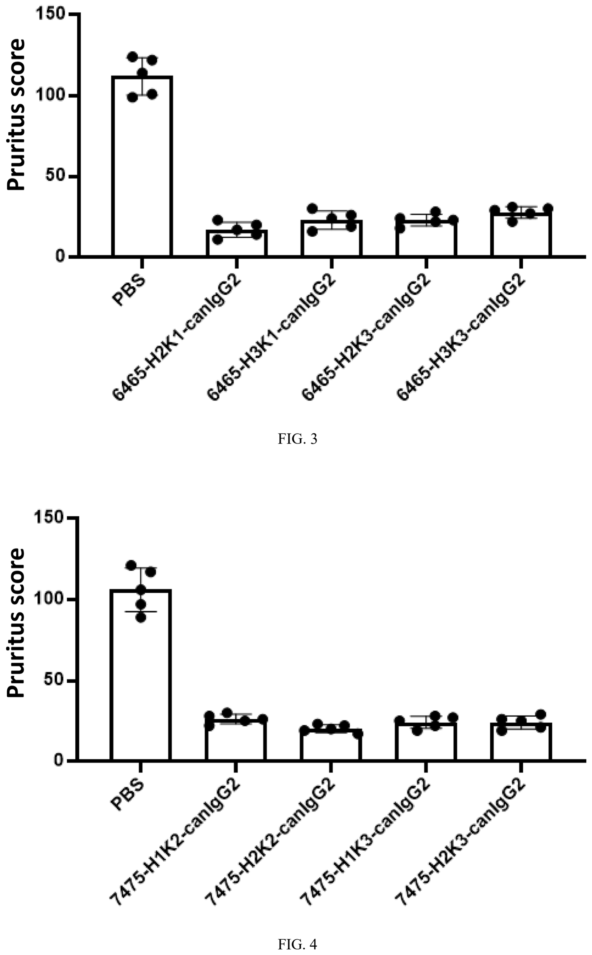

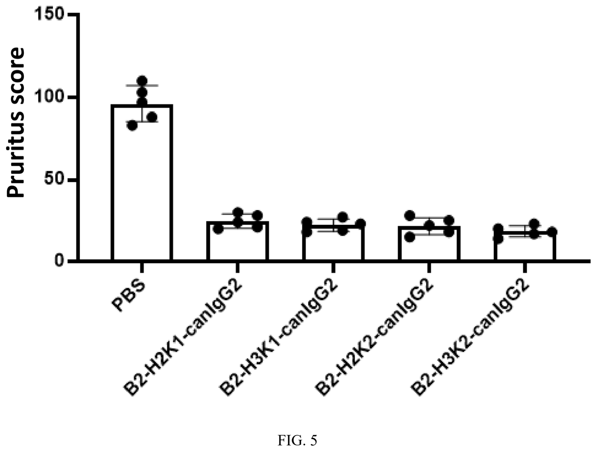

The same experimental method was used for evaluating the efficacy of 6465, 7475 and B2 canine-derived antibodies (6465-H2K1-canIgGB, 6465-H3K1-canIgGB, 6465-H2K3-canIgGB, 6465-H3K3-canIgGB, 7475-H1K2-canIgGB, 7475-H2K2-canIgGB, 7475-H1K3-canIgGB, 7475-H2K3-canIgGB, B2-H2K1-canIgGB, B2-H3K1-canIgGB, B2-H2K2-canIgGB, B2-H3K2-canIgGB), and the results were shown in FIG. 3, FIG. 4 and FIG. 5 respectively. It was indicated that, similar to the chimeric antibodies, the 12 canine-derived antibodies had an obviously inhibitory effect, and the pruritus score during the four-hour observation period after modeling was decreased by more than 75%, the performance of the other canine-derived antibodies shown in Table 1 was similar to that of the 12 canine-derived antibodies.

In addition, the therapeutic efficacy of the anti-canine IL-31canine-derived antibodies was evaluated in a flea-induced allergic dermatitis model, and preliminary results showed that canine-derived antibodies could also significantly inhibit flea-induced pruritus behavior. The therapeutic efficacy results on dogs with atopic dermatitis also showed that the canine-derived antibodies could reduce the pruritus symptoms of dogs with atopic dermatitis.

The adverse reactions were not found in the experimental dogs during the above-mentioned test period and the subsequent one month.

The above results indicate that the antibodies constructed by the present disclosure have high affinity and high activity, and can inhibit canine pruritic skin diseases caused by canine IL-31 and fleas by blocking the binding of canine IL-31 with its receptor, and can reduce the pruritus symptoms of dogs with atopic dermatitis.

The above content describes in detail the preferred embodiments of the present disclosure, but the present disclosure is not limited thereto. A variety of simple modifications can be made in regard to the technical solutions of the present disclosure within the scope of the technical concept of the present disclosure, including a combination of individual technical features in any other suitable manner, such simple modifications and combinations thereof shall also be regarded as the content disclosed by the present disclosure, each of them falls into the protection scope of the present disclosure.

In the present disclosure, the sequences of SEQ ID NOs: 1-39 are as follows, wherein SEQ ID NOs: 29, 34 and 39 are too short to be recognized by the software WIPO Sequence, thus the "intentionally skipped treatment" is performed in the software, but the treatment does not affect the retrieval, and the original sequences of SEQ ID NOs: 1-39 are referred to as the original disclosure.
SEQ ID NO: 1 is NYYLY
SEQ ID NO: 2 is ELNPRNGGTNLNAKFKT
SEQ ID NO: 3 is GGTTVVVRDVMDF
SEQ ID NO: 4 is RASESVDSYGNSFMH
SEQ ID NO: 5 is RASNLES
SEQ ID NO: 6 is QQSNEDPYT
SEQ ID NO: 7 is SYAMS
SEQ ID NO: 8 is YISNGGDYIFYADTVKG
SEQ ID NO: 9 is RGHYNTSSYWYFDV
SEQ ID NO: 10 is KASENVGTYVS
SEQ ID NO: 11 is GASNRYT
SEQ ID NO: 12 is GQSYSYPPT
SEQ ID NO: 13 is DAWMD
SEQ ID NO: 14 is EIRSRAESHATYYAESVKG
SEQ ID NO: 15 is ASTMITTGWFAY
SEQ ID NO: 16 is RASQNISVYLH
SEQ ID NO: 17 is YVSQSIS
SEQ ID NO: 18 is QNGHSFPYT
SEQ ID NO: 19 is GYSFTNY
SEQ ID NO: 20 is NPRNGG
SEQ ID NO: 21 is GFTFSSY
SEQ ID NO: 22 is SNGGDY
SEQ ID NO: 23 is GFTFSDA
SEQ ID NO: 24 is RSRAESHA
SEQ ID NO: 25 is GYSFTNYY
SEQ ID NO: 26 is LNPRNGGT
SEQ ID NO: 27 is TRGGTTVVVRDVMDF
SEQ ID NO: 28 is ESVDSYGNSF
SEQ ID NO: 29 is RA
SEQ ID NO: 30 is GFTFSSYA
SEQ ID NO: 31 is ISNGGDYI
SEQ ID NO: 32 is TRRGHYNTSSYWYFDV
SEQ ID NO: 33 is ENVGTY
SEQ ID NO: 34 is GA
SEQ ID NO: 35 is GFTFSDAW
SEQ ID NO: 36 is IRSRAESHAT
SEQ ID NO: 37 is TPASTMITTGWFAY
SEQ ID NO: 38 is QNISVY
SEQ ID NO: 39 is YV

## Claims

1. An antibody capable of binding to interleukin-31 or an antigen binding fragment thereof, wherein the amino acid sequence of heavy chain variable region CDR1 of the antibody or the antigen-binding fragment thereof has identity no lower than 80% with the amino acid sequence as set forth as SEQ ID NO: 1, 7 or 13, the amino acid sequence of heavy chain variable region CDR2 has identity no lower than 80% with the amino acid sequence as set forth as SEQ ID NOs: 2, 8 and 14, the amino acid sequence of heavy chain variable region CDR3 has identity no lower than 80% with the amino acid sequence as set forth as SEQ ID NOs: 3, 9 and 15, the amino acid sequence of light chain variable region CDR1 has identity no lower than 80% with the amino acid sequence as set forth as SEQ ID NOs: 4, 10 and 16, the amino acid sequence of light chain variable region CDR2 has identity no lower than 80% with the amino acid sequence as set forth as SEQ ID NOs: 5, 11 and 17, and the amino acid sequence of light chain variable region CDR3 has identity no lower than 80% with the amino acid sequence as set forth as SEQ ID NOs: 6, 12 and 18.

2. The antibody or the antigen binding fragment thereof according to claim 1, wherein the number of amino acid residues in the heavy chain variable region CDR1 does not exceed 10;
and/or, the number of amino acid residues in the heavy chain variable region CDR2 does not exceed 22;
and/or, the number of amino acid residues in the heavy chain variable region CDR3 does not exceed 17;
and/or, the number of amino acid residues in the light chain variable region CDR1 does not exceed 18;
and/or, the number of amino acid residues in the light chain variable region CDR2 does not exceed 10;
and/or, the number of amino acid residues in the light chain variable region CDR3 does not exceed 12.

3. The antibody or the antigen binding fragment thereof according to claim 1, wherein the amino acid sequence of the heavy chain variable region CDR1 is as set forth as SEQ ID NO: 1, the amino acid sequence of the heavy chain variable region CDR2 is as set forth as SEQ ID NO: 2, the amino acid sequence of the heavy chain variable region CDR3 is as set forth as SEQ ID NO: 3, the amino acid sequence of the light chain variable region CDR1 is as set forth as SEQ ID NO: 4, the amino acid sequence of the light chain variable region CDR2 is as set forth as SEQ ID NO: 5, and the amino acid sequence of the light chain variable region CDR3 is as set forth as SEQ ID NO: 6;
or, the amino acid sequence of the heavy chain variable region CDR1 is as set forth as SEQ ID NO: 7, the amino acid sequence of the heavy chain variable region CDR2 is as set forth as SEQ ID NO: 8, the amino acid sequence of the heavy chain variable region CDR3 is as set forth as SEQ ID NO: 9, the amino acid sequence of the light chain variable region CDR1 is as set forth as SEQ ID NO: 10, the amino acid sequence of the light chain variable region CDR2 is as set forth as SEQ ID NO: 11, and the amino acid sequence of the light chain variable region CDR3 is as set forth as SEQ ID NO: 12;
or, the amino acid sequence of the heavy chain variable region CDR1 is as set forth as SEQ ID NO: 13, the amino acid sequence of the heavy chain variable region CDR2 is as set forth as SEQ ID NO: 14, the amino acid sequence of the heavy chain variable region CDR3 is as set forth as SEQ ID NO: 15, the amino acid sequence of the light chain variable region CDR1 is as set forth as SEQ ID NO: 16, the amino acid sequence of the light chain variable region CDR2 is as set forth as SEQ ID NO: 17, and the amino acid sequence of the light chain variable region CDR3 is as set forth as SEQ ID NO: 18;
or, SEQ ID NO: 1 is substituted with SEQ ID NO: 19 or 25; SEQ ID NO: 7 is substituted with SEQ ID NO: 21 or 30, SEQ ID NO: 13 is substituted with SEQ ID NO: 23 or 35, SEQ ID NO: 2 is substituted with SEQ ID NO: 20 or 26, SEQ ID NO: 8 is substituted with SEQ ID NO: 22 or 31, SEQ ID NO: 14 is substituted with SEQ ID NO: 24 or 36, SEQ ID NO: 3 is substituted with SEQ ID NO: 27, SEQ ID NO: 9 is substituted with SEQ ID NO: 32, SEQ ID NO: 15 is substituted with SEQ ID NO: 37, SEQ ID NO: 4 is substituted with SEQ ID NO: 28, SEQ ID NO: 10 is substituted with SEQ ID NO: 33, SEQ ID NO: 16 is substituted with SEQ ID NO: 38, SEQ ID NO: 5 is substituted with SEQ ID NO: 29 (amino acid sequence is RA), SEQ ID NO: 11 is substituted with SEQ ID NO: 34 (amino acid sequence is GA), SEQ ID NO: 17 is substituted with SEQ ID NO: NO: 39 (amino acid sequence is YV).

4. The antibody or the antigen binding fragment thereof according to any one of claims 1-3, wherein the antibody is a chimeric antibody;
preferably, the antibody heavy chain variable region is selected from SEQ ID NOS: 40, 48, 56, and the antibody light chain variable region is selected from SEQ ID NOS: 44, 52, 60;
preferably, the amino acid sequence of the heavy chain variable region is SEQ ID NO: 40, and the amino acid sequence of the light chain variable region is SEQ ID NO: 44;
or, the amino acid sequence of the heavy chain variable region is SEQ ID NO: 48, and the amino acid sequence of the light chain variable region is SEQ ID NO: 52;
or, the amino acid sequence of the heavy chain variable region is SEQ ID NO: 56, and the amino acid sequence of the light chain variable region is SEQ ID NO: 60;
preferably, the heavy chain amino acid sequence is selected from SEQ ID NO: 66, 74 or 82; the light chain amino acid sequence is selected from SEQ ID NO: 70, 78 or 86;
more preferably, the antibody may have a heavy chain as set forth as SEQ ID NO: 66 and a light chain as set forth as SEQ ID NO: 70, or a heavy chain as set forth as SEQ ID NO: 74 and a light chain as set forth as SEQ ID NO: 78, or a heavy chain as set forth as SEQ ID NO: 82 and a light chain as set forth as SEQ ID NO: 86.

5. The antibody or the antigen binding fragment thereof according to any one of claims 1-3, wherein the antibody is a canine-derived antibody;
preferably, the heavy chain variable region framework FR amino acid sequence is selected from canine germline heavy chain sequences, preferably having 0-5 reverse mutation residues;
preferably, the light chain variable region framework FR amino acid sequence is selected from canine germline light chain sequences, preferably having 0-5 reverse mutation residues;
preferably, the amino acid sequence of the heavy chain variable region is one of the amino acid sequences as set forth as SEQ ID NOs: 41-43, 49-51 and 57-59, and the amino acid sequence of the light chain variable region is one of the amino acid sequences as set forth as SEQ ID NOs: 45-47, 53-55 and 61-63;
preferably, the amino acid sequence of the heavy chain variable region is one of the amino acid sequences as set forth as SEQ ID NOs: 41-43, and the amino acid sequence of the light chain variable region is one of the amino acid sequences as set forth as SEQ ID NOs: 45-47; more preferably, the amino acid sequence of the heavy chain variable region is one of the amino acid sequences as set forth as SEQ ID NOs: 42 and 43, and the amino acid sequence of the light chain variable region is one of the amino acid sequences as set forth as SEQ ID NOs: 45 and 47;
preferably, the amino acid sequence of the heavy chain variable region is one of the amino acid sequences as set forth as SEQ ID NOs: 49-51, and the amino acid sequence of the light chain variable region is one of the amino acid sequences as set forth as SEQ ID NOs: 53-55; more preferably, the amino acid sequence of the heavy chain variable region is one of the amino acid sequences as set forth as SEQ ID NOs: 49 and 50, and the amino acid sequence of the light chain variable region is one of the amino acid sequences as set forth as SEQ ID NOs: 54 and 55;
preferably, the amino acid sequence of the heavy chain variable region is one of the amino acid sequences as set forth as SEQ ID NOs: 57-59, and the amino acid sequence of the light chain variable region is one of the amino acid sequences as set forth as SEQ ID NOs: 61-63; more preferably, the amino acid sequence of the heavy chain variable region is one of the amino acid sequences as set forth as SEQ ID NOs: 58 and 59, and the amino acid sequence of the light chain variable region is one of the amino acid sequences as set forth as SEQ ID NOs: 61 and 62;
preferably, the amino acid sequence of the heavy chain is one of the amino acid sequences as set forth as SEQ ID NOs: 67-69, 75-77 and 83-85;
preferably, the amino acid sequence of the light chain is one of the amino acid sequences as set forth as SEQ ID NOs: 71-73, 79-81 and 87-89;
more preferably, the antibody may be composed of a heavy chain with an amino acid sequence as set forth as SEQ ID NO: 67 and a light chain with an amino acid sequence as set forth as SEQ ID NO: 71, or a heavy chain with an amino acid sequence as set forth as SEQ ID NO: 68 and a light chain with an amino acid sequence as set forth as SEQ ID NO: 71, or a heavy chain with an amino acid sequence as set forth as SEQ ID NO: 69 and a light chain with an amino acid sequence as set forth as SEQ ID NO: 71, or a heavy chain with an amino acid sequence as set forth as SEQ ID NO: 67 and a light chain with an amino acid sequence as set forth as SEQ ID NO: 72, or a heavy chain with an amino acid sequence as set forth as SEQ ID NO: 68 and a light chain with an amino acid sequence as set forth as SEQ ID NO: 72, or a heavy chain with an amino acid sequence as set forth as SEQ ID NO: 69 and a light chain with an amino acid sequence as set forth as SEQ ID NO: 72, or a heavy chain with an amino acid sequence as set forth as SEQ ID NO: 67 and a light chain with an amino acid sequence as set forth as SEQ ID NO: 73, or a heavy chain with an amino acid sequence as set forth as SEQ ID NO: 68 and a light chain with an amino acid sequence as set forth as SEQ ID NO: 73, or a heavy chain with an amino acid sequence as set forth as SEQ ID NO: 69 and a light chain with an amino acid sequence as set forth as SEQ ID NO: 73, or a heavy chain with an amino acid sequence as set forth as SEQ ID NO: 75 and a light chain with an amino acid sequence as set forth as SEQ ID NO: 79, or a heavy chain with an amino acid sequence as set forth as SEQ ID NO: 76 and a light chain with an amino acid sequence as set forth as SEQ ID NO: 79, or a heavy chain with an amino acid sequence as set forth as SEQ ID NO:77 and a light chain with an amino acid sequence as set forth as SEQ ID NO:79, or a heavy chain with an amino acid sequence as set forth as SEQ ID NO:75 and a light chain with an amino acid sequence as set forth as SEQ ID NO:80, or a heavy chain with an amino acid sequence as set forth as SEQ ID NO:76 and a light chain with an amino acid sequence as set forth as SEQ ID NO:80, or a heavy chain with an amino acid sequence as set forth as SEQ ID NO:77 and a light chain with an amino acid sequence as set forth as SEQ ID NO:80, or a heavy chain with an amino acid sequence as set forth as SEQ ID NO:75 and a light chain with an amino acid sequence as set forth as SEQ ID NO:81, or a heavy chain with an amino acid sequence as set forth as SEQ ID NO:76 and a light chain with an amino acid sequence as set forth as SEQ ID NO:81, or a heavy chain with an amino acid sequence as set forth as SEQ ID NO:77 and a light chain with an amino acid sequence as set forth as SEQ ID NO:81, or a heavy chain with an amino acid sequence as set forth as SEQ ID NO:83 and a light chain with an amino acid sequence as set forth as SEQ ID NO:87, or a heavy chain with an amino acid sequence as set forth as SEQ ID NO:84 and a light chain with an amino acid sequence as set forth as SEQ ID NO:87, or a heavy chain with an amino acid sequence as set forth as SEQ ID NO:85 and a light chain with an amino acid sequence as set forth as SEQ ID NO:87, or a heavy chain with an amino acid sequence as set forth as SEQ ID NO:83 and a light chain with an amino acid sequence as set forth as SEQ ID NO: 88, or a heavy chain with an amino acid sequence as set forth as SEQ ID NO:84 and a light chain with an amino acid sequence as set forth as SEQ ID NO:88, or a heavy chain with an amino acid sequence as set forth as SEQ ID NO:85 and a light chain with an amino acid sequence as set forth as SEQ ID NO:88, or a heavy chain with an amino acid sequence as set forth as SEQ ID NO:83 and a light chain with an amino acid sequence as set forth as SEQ ID NO:89, or a heavy chain with an amino acid sequence as set forth as SEQ ID NO:84 and a light chain with an amino acid sequence as set forth as SEQ ID NO:89, or a heavy chain with an amino acid sequence as set forth as SEQ ID NO:85 and a light chain with an amino acid sequence as set forth as SEQ ID NO:89;
preferably, the antibody or the antigen binding fragment thereof is at least one selected from the group consisting of a monoclonal antibody, a polyclonal antibody, a multimeric antibody and a CDR grafting antibody;
preferably, the antibody or the antigen binding fragment thereof is one selected from the group consisting of a single-chain antibody, a Fab antibody, a Fv antibody, a single-domain antibody, a (Fab)2 fragment, a scFv-Fc fusion protein, a scFv-Fv fusion protein, a Fv fragment and a minimum antigen recognition unit.

6. A nucleotide molecule, wherein the nucleotide molecule encodes the antibody or the antigen binding fragment thereof according to any one of claims 1-5;
preferably, the nucleotide sequence of the DNA molecule encoding the heavy chain is one of SEQ ID NOS: 90-93, 98-101 and 106-109;
preferably, the nucleotide sequence of the DNA molecule encoding the light chain is one of SEQ ID NOS: 94-97, 102-105 and 110-113;
more preferably, the DNA encoding the antibody comprises: the heavy chain as set forth as SEQ ID NO: 90, and the light chain as set forth as SEQ ID NO: 94; the heavy chain as set forth as SEQ ID NO: 91, and the light chain as set forth as SEQ ID NO: 95; the heavy chain as set forth as SEQ ID NO: 92, and the light chain as set forth as SEQ ID NO: 95; the heavy chain as set forth as SEQ ID NO: 93, and the light chain as set forth as SEQ ID NO: 95; the heavy chain as set forth as SEQ ID NO: 91, and the light chain as set forth as SEQ ID NO: 96; the heavy chain as set forth as SEQ ID NO: 92, and the light chain as set forth as SEQ ID NO: 96; the heavy chain as set forth as SEQ ID NO: 93, and the light chain as set forth as SEQ ID NO: 96; the heavy chain as set forth as SEQ ID NO: 91, and the light chain as set forth as SEQ ID NO: 97; the heavy chain as set forth as SEQ ID NO: 92, and the light chain as set forth as SEQ ID NO: 97; the heavy chain as set forth as SEQ ID NO: 93, and the light chain as set forth as SEQ ID NO: 97; the heavy chain as set forth as SEQ ID NO: 98, and the light chain as set forth as SEQ ID NO: 102; the heavy chain as set forth as SEQ ID NO: ID NO:99, the light chain as set forth as SEQ ID NO:103; the heavy chain as set forth as SEQ ID NO:100, the light chain as set forth as shown in SEQ ID NO:103; the heavy chain as set forth as SEQ ID NO:101, the light chain as set forth as SEQ ID NO:103; the heavy chain as set forth as SEQ ID NO:99, the light chain as set forth as SEQ ID NO:104; the heavy chain as set forth as SEQ ID NO:100, the light chain as set forth as SEQ ID NO:104; the heavy chain as set forth as SEQ ID NO:101, the light chain as set forth as SEQ ID NO:104; the heavy chain as set forth as SEQ ID NO:99, the light chain is shown as set forth as SEQ ID NO:105; the heavy chain as set forth as SEQ ID NO:100, the light chain as set forth as SEQ ID NO:105; the heavy chain as set forth as SEQ ID NO:101, the light chain as set forth as SEQ ID NO:105; the heavy chain as set forth as SEQ ID NO:106, the light chain as set forth as SEQ ID NO:110; the heavy chain as set forth as SEQ ID NO:107, the light chain as set forth as SEQ ID NO:111; the heavy chain as set forth as SEQ ID NO: 108, and the light chain as set forth as SEQ ID NO: 111; the heavy chain as set forth as SEQ ID NO: 109, and the light chain as set forth as SEQ ID NO: 111; the heavy chain as set forth as SEQ ID NO: 107, and the light chain as set forth as SEQ ID NO: 112; the heavy chain as set forth as SEQ ID NO: 108, and the light chain as set forth as SEQ ID NO: 112; the heavy chain as set forth as SEQ ID NO: 109, and the light chain as set forth as SEQ ID NO: 112; the heavy chain as set forth as SEQ ID NO: 107, and the light chain as set forth as SEQ ID NO: 113; the heavy chain as set forth as SEQ ID NO: 108, and the light chain as set forth as SEQ ID NO: 113; the heavy chain as set forth as SEQ ID NO: 109, and the light chain as set forth as SEQ ID NO: 113.

7. An expression vector, wherein the expression vector expresses the nucleotide molecule according to claim 6.

8. A method for preparing an antibody or an antigen binding fragment thereof, wherein the method comprises transforming a host cell with the expression vector according to claim 7;
preferably, the host cell is a mammalian cell, more preferably a CHOK1 cell or a HEK293 cell.

9. A pharmaceutical composition, wherein the pharmaceutical composition comprises an excipient and the antibody or antigen binding fragment thereof according to any one of claims 1-5, or the nucleotide molecule according to claim 6, or the expression vector according to claim 7;
preferably, the excipient is at least one selected from the group consisting of a pharmaceutically acceptable carrier, a pharmaceutically acceptable excipient and an adjuvant.

10. A method for treating a pruritic skin disease in a mammal, wherein the method comprises:
administering to the mammal the antibody or the antigen binding fragment thereof according to any one of claims 1-5, or the nucleic acid molecule according to claim 6, or the expression vector according to claim 7, or the pharmaceutical composition according to claim 9;
preferably, the antibody or the antigen binding fragment thereof is administered in an amount of 1-5 mg/kg;
more preferably, the administration method is at least one selected from the group consisting of intravenous injection, intramuscular injection, subcutaneous injection, sublingual administration, rectal perfusion, inhalation administration, and spray administration, more preferably subcutaneous injection;
further preferably, the pruritic skin disease is a disease associated with abnormal expression of IL-31;
further preferably, the pruritic skin disease is at least one selected from the group consisting of atopic dermatitis, contact dermatitis, nodular pruritus and eczema;
more preferably, the mammal is at least one selected from the group consisting of animals of *Felidae, Canidae, Suidae, Bovidae, Caprinae, Cercopithecidae, Muridae* and *Equidae* family;
preferably, the mammal is at least one selected from the group consisting of a canine, a wolf, a jackal and a fox, more preferably a canine.

11. Use of the antibody or the antigen binding fragment thereof according to any one of claims 1-5, or the nucleic acid molecule according to claim 6, or the expression vector according to claim 7 in the preparation of a medicament for treating a pruritic skin disease in a mammal;
preferably, the pruritic skin disease is a lesion associated with abnormal expression of IL-31;
further preferably, the pruritic skin disease is at least one selected from the group consisting of atopic dermatitis, contact dermatitis, nodular pruritus and eczema;
more preferably, the mammal is at least one selected from the group consisting of animals of *Felidae, Canidae, Suidae, Bovidae, Caprinae, Cercopithecidae, Muridae* and *Equidae* family;
preferably, the mammal is at least one selected from the group consisting of a canine, a wolf, a jackal and a fox, more preferably a canine.
